# EUROPÄISCHE PATENTANMELDUNG

(11) **EP 3 058 966 A1**
(43) Veröffentlichungstag der Anmeldung: **24.08.2016**
(21) Anmeldenummer: 15155890.5
(22) Anmeldetag: 20.02.2015
(51) Int. Cl.: A61M 1/06

(54) **Brusthaube**

(71) Anmelder: Medela Holding AG, 6340 Baar (CH)
(72) Erfinder: Rigert, Mario, 6033 Buchrain (CH); Käppeli, Daniela, 6300 Zug (CH); Schlienger, André, 8933 Maschwanden (CH)
(74) Vertreter: Clerc, Natalia

(57) **Zusammenfassung**

Eine Brusthaube für eine Brustpumpe zum Abpumpen von menschlicher Muttermilch weist einen Trichter (11) zur Auflage auf die menschliche Mutterbrust und ein Anschlussteil (10) auf, wobei sich der Trichter (11) zur Mutterbrust hin erweitert und mindestens eine Brustwarze und eine die Brustwarze umgebende Areola der Mutterbrust umschliesst. Der Trichter (11) weist ein brustnahes Ende auf, welches eine Auflagekante (12) zur Kontaktierung der Mutterbrust bildet. Die Brusthaube weist einen Durchgang (14) auf, welcher sich vom brustnahen Ende des Trichters (11) bis zum brustfernen Ende des Anschlussteils (10) erstreckt und welcher zum Anlegen eines Unterdrucks an die Mutterbrust und zum Abfliessen abgepumpter Muttermilch dient. Die Auflagekante (12) des Trichters (11) ist durch eine nicht in einer Ebene liegende Raumkurve gebildet, wobei der Trichter (12) mindestens zwei Symmetrieebenen (S₁, S₂) aufweist. Die erfindungsgemässe Brusthaube ermöglicht ein optimal dichtendes und für die Mutter angenehmes Anliegen an die Mutterbrust.

## Beschreibung

### TECHNISCHES GEBIET

Die vorliegende Erfindung betrifft eine Brusthaube für eine Brustpumpe zum Abpumpen von menschlicher Muttermilch.

### STAND DER TECHNIK

Brustpumpensysteme zum Abpumpen von menschlicher Muttermilch sind wohlbekannt. Sie weisen eine manuell oder elektromotorisch betriebene Vakuumpumpe, mindestens eine Brusthaube zur Auflage auf die Mutterbrust, einen Adapter und einen Milchsammelbehälter auf, in welchem die abgepumpte Milch aufgefangen wird. Die Brusthaube ist mit der Vakuumpumpe direkt oder über eine Saugleitung verbunden, so dass in der Brusthaube ein zyklisch variierender Unterdruck an die Brust angelegt werden kann, um die Milch aus der Brust abzupumpen. Der Adapter hält die Brusthaube und verbindet sie mit der Vakuumpumpe bzw. der Saugleitung. Der Adapter ermöglicht zudem die Verbindung mit dem Milchsammelbehälter, entweder durch eine direkte Kopplung des Behälters an den Adapter oder über eine Milchleitung.

Bekannte Brusthauben weisen einen Trichter und ein Anschlussteil zur Verbindung mit einem Adapter auf. Üblicherweise sind die Trichter kegelstumpfförmig ausgebildet, wobei sie steif oder weich sein können. Steife Trichter können zudem mit einer weichen Einlage versehen sein.

Der Trichter sollte möglichst dicht an der Mutterbrust anliegen, damit eine wirkungsvolle Pumpkammer durch Mutterbrust und Brusthaube gebildet werden kann. Zudem sollte er angenehm auf der Mutterbrust aufliegen, um ein möglichst druckstellenfreies und entspanntes Abpumpen zu ermöglichen. Aus dem Stand der Technik sind verschiedene Brusthauben bekannt, welche diese Ziele zu erreichen versuchen.

WO 2011/035488 offenbart eine relativ kleine, sehr flexible Brusthaube, deren Öffnungswinkel sich der Brust anpasst.

US 6 673 037 zeigt eine steife Brusthaube mit einem Trichter, welcher einen elliptischen Grundriss aufweist. Auf der Innenseite des Trichters sind Erhebungen vorhanden, welche auf die Brust einwirken. US 4 772 262 offenbart eine weiche Brusthaube mit einer elliptischen Öffnung.

US 7 413 557 beschreibt eine Brusthaube mit einem asymmetrischen Trichter, welcher eine Oberseite zur Anlage auf der oberen Seite der Brust und eine Unterseite zur Anlage auf der unteren Seite der Brust aufweist. Diese Brusthaube weist den Nachteil auf, dass sie für kleine Brüste zu gross ist und bei speziell geformten Brüsten ebenfalls nicht optimal anliegt. Auch die Brusthaube gemäss GB 2 138 686 ist asymmetrisch geformt.

US 2006/0116632 schlägt vor, das Anschlussteil der Brusthaube in einem Winkel zum kegelförmigen Trichter anzuordnen.

Die Grösse und Form der menschlichen Mutterbrust ist sehr stark vom Individuum abhängig und entsprechend unterschiedlich. Zudem weist die Mutterbrust üblicherweise nicht eine perfekte Kegelform auf. Der Stand der Technik hat sich deshalb, insbesondere im Bereich der steifen und halbsteifen Trichter, mit Kompromisslösungen begnügt oder Brusthauben in unterschiedlichen Grössen angeboten.

US 6 387 072 und US 6 723 066 offenbaren zum Beispiel ein Brusthaubenset mit einer steifen Haubenbasis und einem Set von verschieden grossen, in die Basis einsetzbaren Brusthauben. Die Trichter der Brusthauben sind kegelstumpfförmig ausgebildet, wobei in einer Ausführungsform der umlaufende Rand eine Ausnehmung aufweist. Diese Brusthaube kann so auf die Brust gelegt werden, dass die Ausnehmung über einen empfindlichen oder anatomisch speziell geformten Bereich der Mutterbrust zu liegen kommt und dieser Bereich somit geschont wird.

### DARSTELLUNG DER ERFINDUNG

Es ist deshalb eine Aufgabe der Erfindung, eine Brusthaube zu schaffen, welche bestmöglich dichtend an der Mutterbrust anliegt.

Diese Aufgabe löst eine Brusthaube mit den Merkmalen des Patentanspruchs 1 sowie mit den Merkmalen des Patentanspruchs 15.

In einer Ausführungsform weist die erfindungsgemässe Brusthaube für eine Brustpumpe zum Abpumpen von menschlicher Muttermilch einen Trichter zur Auflage auf die menschliche Mutterbrust und ein Anschlussteil auf, wobei sich der Trichter zur Mutterbrust hin erweitert, um mindestens eine Brustwarze und eine die Brustwarze umgebende Areola der Mutterbrust zu umschliessen. Der Trichter weist ein brustnahes Ende auf, welches eine Auflagekante zur Kontaktierung der Mutterbrust bildet. Die Brusthaube weist einen Durchgang oder Kanal auf, welcher sich vom brustnahen Ende des Trichters bis zum brustfernen Ende des Anschlussteils erstreckt und welcher zum Anlegen eines Unterdrucks an die Mutterbrust und zum Abfliessen abgepumpter Muttermilch dient. Die Auflagekante des Trichters ist durch eine nicht in einer Ebene liegende Raumkurve gebildet und der Trichter weist mindestens zwei Symmetrieebenen auf.

Alternativ oder zusätzlich zu den mindestens zwei Symmetrieebenen ist der Trichter ein gerader Kegelstumpf. Vorzugsweise ist er ein gerader Kreiskegelstumpf. Er kann beispielsweise auch ein gerader Kegelstumpf mit elliptischem Querschnitt sein.

Die erfindungsgemässe Brusthaube liegt dank ihrer speziellen räumlichen Kurvenform optimal an der Brust auf. Die spezielle Form ermöglicht es, die Brusthaube in verschiedenen Drehpositionen oder Ausrichtungen an die Brust zu legen. Die Mutter kann die für sie und ihre Brustform optimale Drehposition wählen. "Optimal" ist in diesem Text im Sinne von bestmöglich dicht und bestmöglich schonend und sanft zu verstehen.

Des Weiteren wirkt die Brusthaube mit der räumlichen Kurvenform ergonomischer und femininer. Allfällige psychologische Hürden der Mutter werden dadurch abgebaut, was wiederum den Erfolg beim Abpumpen erhöht.

Vorzugsweise weist nicht nur der Trichter, sondern auch die Auflagekante selber mindestens zwei Symmetrieebenen auf.

Es hat sich gezeigt, dass die Brusthaube optimal geformt ist, wenn der Trichter und/oder die Auflagekante genau zwei Symmetrieebenen aufweist.

Vorzugsweise ist der Trichter ein gerader Kegelstumpf, wobei das brustseitige Ende des Kegelstumpfes die Auflagekante bildet. Ist er ein gerader Kreiskegelstumpf, ist verhindert, dass die Brustwarze beim Abpumpen die Flanke des Trichters berührt.

In bevorzugten Ausführungsbeispielen weist eine Projektion der Auflagekante in eine Ebene senkrecht zu einer Längsachse des Trichters die Form einer Ellipse auf. Ist der Trichter ein gerader Kreiskegelstumpf und die Auflagekante in der genannten Projektion eine Ellipse, so ist eine bestmögliche Passform einer steifen Brusthaube erzielt.

Anstelle einer in sich geschlossenen Ellipsenform lässt sich die genannte Projektion der Auflagekante auch in einer anderen Form ausbilden. Beispielsweise kann sie eine beliebige ovale Form aufweisen, d.h. eine ebene konvexe Form mit oder ohne Symmetrieachsen. Sie kann jedoch auch andere in sich geschlossene Formen bilden, beispielsweise kann sie mäanderförmig sein und die Form eines drei- oder vierblättrigen Kleeblatts oder einer Blume aufweisen. N-eckige Polygonzüge, d.h. eckige Formen, sind grundsätzlich ebenfalls möglich, wobei die Ecken vorzugsweise abgerundet sind.

Vorzugsweise ist der Öffnungswinkel des Trichters über seinem Umfang gleichbleibend. Dadurch bestimmt lediglich die Form der Auflagekante die optimale Drehposition der Brusthaube auf der Mutterbrust. Ein Berühren der Brustwarze ist an der inneren Flanke des Trichters verhindert.

Vorzugsweise sind der Trichter und/oder die Auflagekante steif ausgebildet.

Die Brusthaube ist üblicherweise aus einem Kunststoff gefertigt. Die Brusthaube kann ohne weitere Einlagen eingesetzt sein. Sie kann jedoch auch mit Einsätzen versehen sein. Vorzugsweise ist die innere Oberfläche des Trichters frei von Erhebungen und Vertiefungen. Es können jedoch auch weiche, halbharte oder harte Strukturierungen auf der Innenseite des Trichters vorhanden sein, wie sie aus dem Stand der Technik bekannt sind.

Alternativ können der Trichter und/oder die Auflagekante weich oder halbweich ausgebildet sein.

In bevorzugten Ausführungsformen bildet die Auflagekante eine umlaufende Auflagefläche, welche eine Flächenauflage des Trichters auf der Mutterbrust ermöglicht. Die Auflagekante ist somit als Dekompressionskante ausgebildet, welche den Zweck erfüllt, dass es am Umfang des Trichters nicht zu einer Linien- sondern zu einer Flächenauflage auf der Mutterbrust kommt.

Vorzugsweise sind Auflagekante, Trichter und Anschlussteil einstückig ausgebildet. Sie bestehen vorzugsweise aus demselben Material. Das Anschlussteil dient zur Verbindung mit einem Adapter, welcher direkt oder indirekt mit der Brustpumpe und mit dem Milchsammelbehälter verbindbar ist. Anschlussteil und Adapter sind üblicherweise zwei voneinander getrennte Bauteile. Sie können jedoch ebenfalls gemeinsam einstückig ausgebildet sein.

In bevorzugten Ausführungsformen weist der Trichter auf seiner Aussenseite mindestens ein haptisches Element auf, welches sich von der übrigen äusseren Oberfläche des Trichters unterscheidet. Diese haptischen Elemente sind in einer bevorzugten Ausführungsform in sich geschlossene, den Trichter umlaufende Rillen oder Erhebungen. Ihre Projektion in eine Ebene senkrecht zur Längsachse des Trichters ist vorzugsweise identisch mit der Projektion der Raumkurve, insbesondere ist sie vorzugsweise elliptisch. Dank dieser haptischen Elemente wird die Mutter die Vorrichtung intuitiv am Trichter halten. Entsprechen sie zudem der Raumkurve bzw. weisen auf diese hin, dienen sie der Mutter als Orientierungshilfe.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine Seitenansicht einer erfindungsgemässen Brusthaube mit einem Adapter in einer ersten Ausführungsform;
- Figur 2: einen Längsschnitt durch die Vorrichtung gemäss Figur 1;
- Figur 3: eine perspektivische Darstellung der Brusthaube gemäss Figur 1 aus brustseitiger Sicht;
- Figur 4: eine perspektivische Darstellung der Brusthaube gemäss Figur 1 aus brustferner Sicht;
- Figur 5: eine erste Seitenansicht der Brusthaube gemäss Figur 1;
- Figur 6: eine zweite Seitenansicht der Brusthaube gemäss Figur 1;
- Figur 7: eine dritte Seitenansicht der Brusthaube gemäss Figur 1;
- Figur 8: eine brustnahe Ansicht der Brusthaube gemäss Figur 1;
- Figur 9: eine brustferne Ansicht der Brusthaube gemäss Figur 1;
- Figur 10: eine Ansicht der Brusthaube mit zusätzlich eingezeichneten Hilfslinien und Symmetrieebenen;
- Figur 11: eine perspektivische Darstellung einer erfindungsgemässen Brusthaube in einer zweiten Ausführungsform aus brustnaher Sicht;
- Figur 12: eine perspektivische Darstellung der Brusthaube gemäss Figur 11 in einer zweiten Ausführungsform aus brustferner Sicht;
- Figur 13: eine Seitenansicht der Brusthaube gemäss Figur 11 und
- Figur 14: eine brustferne Ansicht der Brusthaube gemäss Figur 11.

### BESCHREIBUNG BEVORZUGTER AUSFÜHRUNGSFORMEN

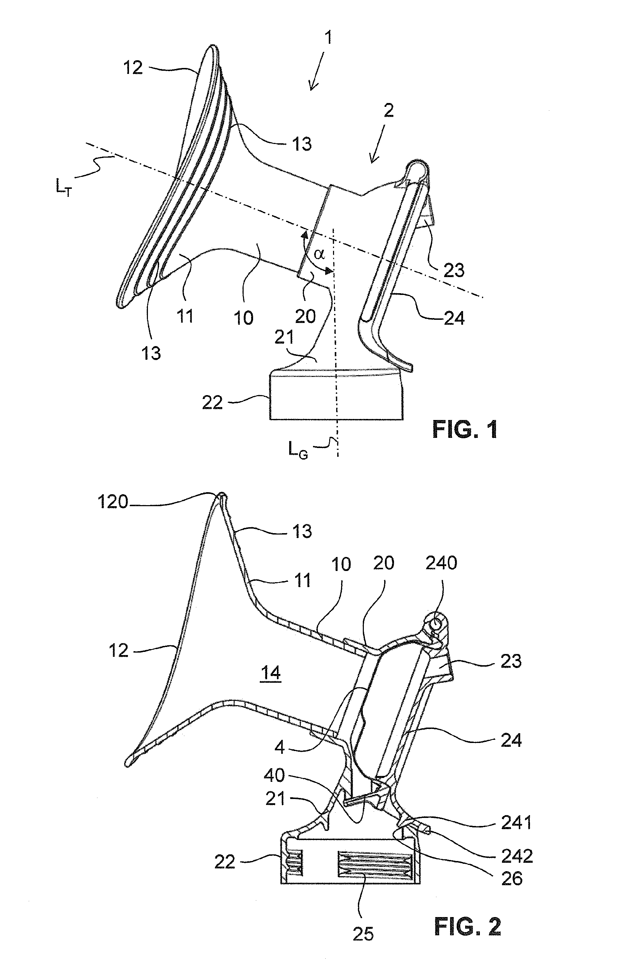

In den Figuren 1 bis 10 ist eine erste Ausführungsform der erfindungsgemässen Brusthaube 1 mit einem Adapter 2 dargestellt. Sie weist ein Anschlussteil 10, hier in Form eines zylinderförmigen Stutzens 10 sowie einen daran einstückig angeformten Trichter 11 auf. Die Brusthaube 1 ist vorzugsweise aus Kunststoff gefertigt. Sie ist vorzugsweise steif ausgebildet.

Die Brusthaube 1 ist mit dem Anschlussteil 10 in einem Aufnahmeteil 20 des Adapters 2 gehalten. Das Aufnahmeteil 20 weist hier die Form eines rohrförmigen Stutzens auf, in welchen das Anschlussteil 10 der Brusthaube 1 einsteckbar ist.

Der Adapter 2 weist einen Hals 21 mit einem Milchanschluss 22 auf. Der Milchanschluss 22 ist wiederum ein Rohrabschnitt, welches ein Innengewinde 25 aufweist zur Befestigung auf einen Milchsammelbehälter auf, insbesondere auf einen Hals einer Milchflasche.

Vorzugsweise ist die Längsmittelachse L_{T} des Trichters 11 in einem Winkel zur Längsmittelsachse L_{G} des Innengewindes 25 und somit des Milchanschlusses 22 ausgerichtet. Der Neigungswinkel α beträgt vorzugsweise 90° bis 120°.

Auf der der Brusthaube 1 abgewandten Seite des Adapters 2 ist ein Vakuumanschluss 23 für einen Vakuumschlauch zur Verbindung mit einer Vakuumpumpe vorhanden. Alternativ kann die Vakuumpumpe direkt an diesem Anschluss angeordnet sein. Zudem kann anstelle einer elektromotorbetriebenen Pumpe auch eine handbetriebene Pumpe mit der erfindungsgemässen Brusthaube verwendet werden.

Die Rückseite des Adapters 2, d.h. die brustferne Seite, ist in diesem Beispiel mit einem Deckel 24 verschlossen.

In Figur 2 ist erkennbar, dass im Adapter 2 eine Medientrennmembran 4 angeordnet ist, welche verhindert, dass abgepumpte Milch von der Brust zum Vakuumanschluss 23 gelangen kann. Dadurch wird eine Verunreinigung der Vakuumpumpe verhindert. Die Medientrennmembran 4 ist flexibel ausgebildet und überträgt den über den Sauganschluss 23 zyklisch angelegten Unter- oder Überdruck in den Trichter 11 der Brusthaube.

An der Medientrennmembran 4 ist eine Lippe angeformt, welche als Ventilklappe 40 eines Rückschlagventils wirkt. Der Sitz des Ventils ist durch den Adapter 2 gebildet. Das Ventil unterbricht die Verbindung zwischen einem Durchgang 14 der Brusthaube 1 und dem Milchanschluss 22, so dass die Verbindung zwischen der Brust und dem Milchsammelbehälter zyklisch unterbrochen ist. Dies begrenzt das Totvolumen im Adapter 2 während des Anlegens des Unterdrucks.

Die Medientrennmembran 4 lässt sich einfach montieren und zwecks Reinigung und/oder Auswechslung entfernen. Das Innere des Adapters 2 ist durch den Deckel 24 auf einfache Art und Weise zugänglich. Der Deckel 24 ist in dieser Ausführungsform schwenkbar gehalten. Die Bezugsziffer 240 bezeichnet das Scharnier des Deckels, die Bezugsziffer 241 eine Rückhaltenase 241, welche in eine Rückhaltekante 26 des restlichen Adapters 2 eingreift. Der Deckel 24 ist mit einem vorstehenden Griff 242 verlängert, damit er einfach angehoben werden kann.

Diese Medientrennmembran 4 und dieser Adapter 2 sind Erfindungen, welche in einer am gleichen Tag wie die vorliegende Patentanmeldung eingereichten Patentanmeldung derselben Anmelderin beschrieben sind. Der Titel der Patentanmeldung lautet "Adapter mit Medientrennmembran für eine Brusthaube". Ihr Inhalt ist hiermit durch Referenz in diesem Text mit aufgenommen.

Anstelle dieses Adapters lässt sich auch ein anderer Adapter oder ein einstückig mit der Brusthaube verbundener Adapter mit der erfindungsgemässen Brusthaube verwenden.

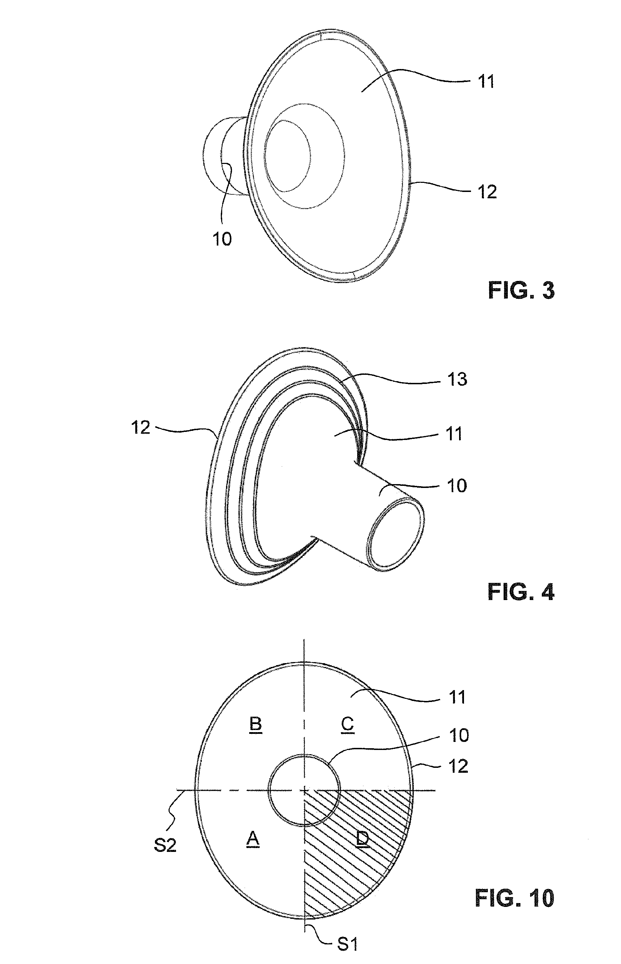

In den Figuren 3 bis 9 ist das erste Ausführungsbeispiel der erfindungsgemässen Brusthaube in mehreren Ansichten und Perspektiven dargestellt. Der Trichter 11 ist kreiskegelstumpfförmig ausgebildet. Er weist einen über seinem Umfang gleichbleibenden Öffnungswinkel γ auf. Der Öffnungswinkel bleibt auch über die axiale Länge des Trichters 11 vorzugsweise konstant. In anderen Ausführungsformen ändert er sich jedoch über die axiale Länge.

Der Trichter 11 erweitert sich zur Brust hin. Sein anschlussteilfernes und somit brustnahes erweiterte Ende bildet eine Auflagekante 12 zur Auflage an die Mutterbrust. Die Auflagekante 12 ist flächig ausgebildet, wie dies in den Figuren 1 und 2 gut erkennbar ist. Diese Auflagefläche 120 dient dazu, dass der Trichter 11 nicht nur linienförmig, sondern flächig auf der Brust aufliegt.

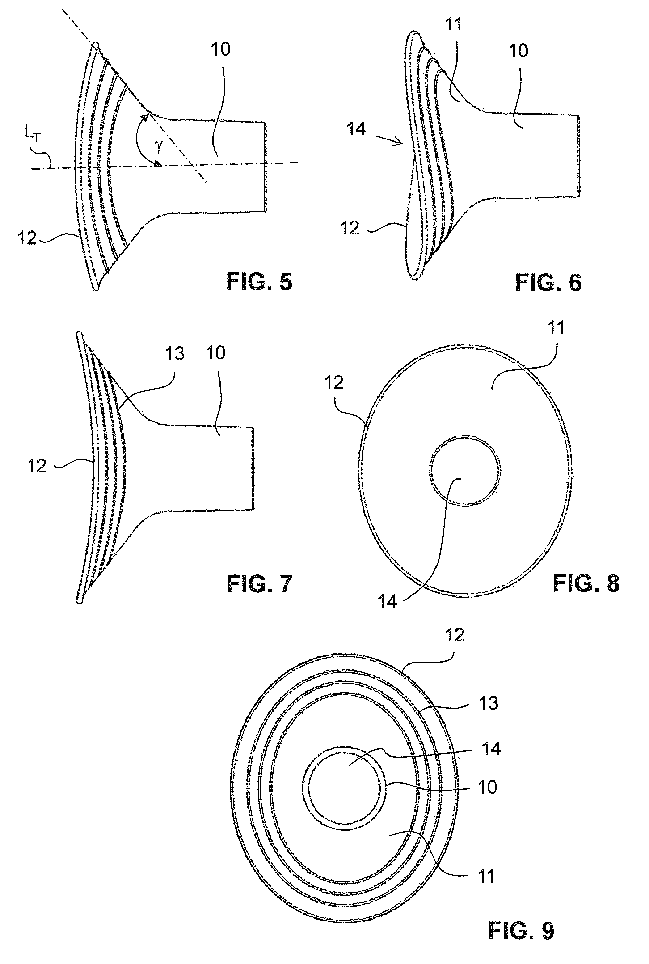

Die Auflagekante 12 ist eine Raumkurve, d.h. sie liegt nicht einer Ebene. Die räumliche Anordnung der Auflagekante 12 ist durch Vergleich der Figuren 5 bis 7 gut erkennbar. Geometrisch gesehen ist der Kreiskegelstumpf nicht in einer Ebene geschnitten, sondern räumlich. Bei geeigneter Wahl der Schnittkurve ergibt sich, wie hier dargestellt, eine Auflagekante 12, deren Projektion in eine Ebene senkrecht zur Längsmittelachse L_{T} des Trichters 11 einer Ellipse entspricht. Dies ist in den Figuren 8 und 9 gut erkennbar. Wie in Figur 10 dargestellt ist, weist diese Projektion zwei Symmetrien auf. Ihre Fläche lässt sich in vier gleich grosse und zueinander bezüglich der Symmetrieachsen S₁ und S₂ spiegelsymmetrische Zonen A, B, C, D aufteilen. Diese zwei Symmetrieachsen S₁ und S₂ sind zudem Teil der zwei Symmetrieebenen, welche senkrecht zur Blattfläche der Zeichnungen verlaufen. Sie bilden die zwei Symmetrieebenen des Trichters 11 und der Auflagefläche 12. Vorzugsweise sind dies die zwei einzigen Symmetrieebenen. Sie sind hier ebenfalls mit dem Bezugszeichen S₁ und S₂ bezeichnet.

Die im Wesentlichen glatte, äussere Oberfläche des Trichters 11 ist mit in sich geschlossenen, umlaufenden Rippen 13 versehen. Die Rippen 13 sind beabstandet zueinander angeordnet und weisen vorzugsweise dieselbe Form auf wie die Auflagekante 12. Wie in Figur 9 erkennbar ist, sind sie im Grundriss elliptisch ausgebildet.

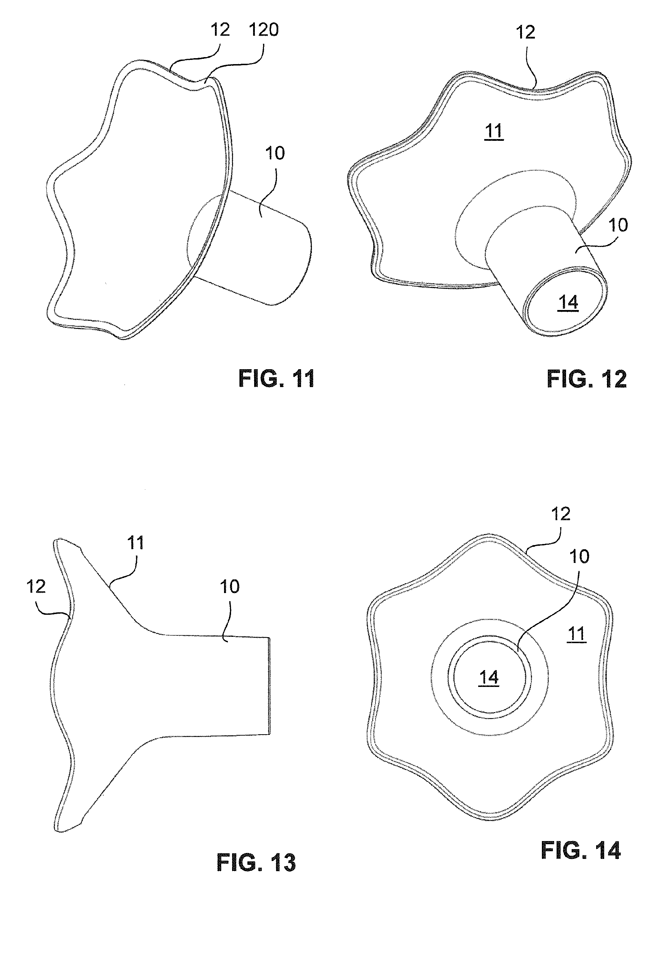

In den Figuren 11 bis 14 ist ein weiteres Ausführungsbeispiel der erfindungsgemässen Brusthaube dargestellt. Sie weist dieselbe Form auf wie die bereits beschriebene Brusthaube. Die Auflagekante 12 ist ebenfalls eine Raumkurve, wie oben beschrieben. Sie ist jedoch mäanderförmig oder geschwungen ausgebildet. Sie bzw. ihre Projektion weist in diesem Beispiel eine blumenähnliche Form auf.

Die erfindungsgemässe Brusthaube ermöglicht ein optimal dichtendes und für die Mutter angenehmes Anliegen an die Mutterbrust.

### BEZUGSZEICHENLISTE

| | | | |
|---|---|---|---|
| 1 | Brusthaube | 25 | Innengewinde |
| 10 | Anschlussteil | 26 | Rückhaltekante |
| 11 | Trichter | | |
| 12 | Auflagekante | 4 | Medientrennmembran |
| 120 | Auflagefläche | 40 | Ventilklappe |
| 13 | haptisches Element | | |
| 14 | Durchgang | A | erste Zone der Brusthaube |
| | | B | zweite Zone der Brusthaube |
| 2 | Adapter | C | dritte Zone der Brusthaube |
| 20 | Aufnahmeteil | D | vierte Zone der Brusthaube |
| 21 | Hals | S₁ | erste Symmetrieebene/achse |
| 22 | Milchanschluss | S₂ | zweite Symmetrieebene/achse |
| 23 | Sauganschluss | L_{T} | Längsmittelachse des Trichters |
| 24 | Deckel | L_{G} | Längsmittelachse des Gewindes |
| 240 | Scharnier | α | Neigungswinkel |
| 241 | Rückhaltenase | γ | Öffnungswinkel |
| 242 | Griff | | |

## Patentansprüche

1. Brusthaube für eine Brustpumpe zum Abpumpen von menschlicher Muttermilch, wobei die Brusthaube einen Trichter (11) zur Auflage auf die menschliche Mutterbrust und ein Anschlussteil (10) aufweist, wobei sich der Trichter (11) zur Mutterbrust hin erweitert, um mindestens eine Brustwarze und eine die Brustwarze umgebende Areola der Mutterbrust zu umschliessen, wobei der Trichter (11) ein brustnahes Ende aufweist, welches eine Auflagekante (12) zur Kontaktierung der Mutterbrust bildet, und wobei die Brusthaube einen Durchgang (14) aufweist, welcher sich vom brustnahen Ende des Trichters (11) bis zum brustfernen Ende des Anschlussteils (10) erstreckt und welcher zum Anlegen eines Unterdrucks an die Mutterbrust und zum Abfliessen abgepumpter Muttermilch dient, **dadurch gekennzeichnet, dass** die Auflagekante (12) des Trichters (11) durch eine nicht in einer Ebene liegende Raumkurve gebildet ist und dass der Trichter (12) mindestens zwei Symmetrieebenen (S₁, S₂) aufweist.

2. Brusthaube nach Anspruch 1, wobei die Auflagekante (12) mindestens zwei Symmetrieebenen (S₁, S₂) aufweist.

3. Brusthaube nach einem der Ansprüche 1 oder 2, wobei der Trichter (11) genau zwei Symmetrieebenen (S₁, S₂) aufweist.

4. Brusthaube nach einem der Ansprüche 1 bis 3, wobei die Auflagekante (12) genau zwei Symmetrieebenen (S₁, S₂) aufweist.

5. Brusthaube nach einem der Ansprüche 1 bis 4, wobei der Trichter (11) ein gerader Kegelstumpf ist und wobei ein brustseitiges Ende des Kegelstumpfes die Auflagekante (12) bildet.

6. Brusthaube nach Anspruch 5, wobei der Kegelstumpf ein Kreiskegelstumpf ist.

7. Brusthaube nach einem der Ansprüche 1 bis 6, wobei eine Projektion der Auflagekante in eine Ebene senkrecht zu einer Längsachse (L_{T}) des Trichters (11) eine Ellipse ist.

8. Brusthaube nach einem der Ansprüche 1 bis 6, wobei eine Projektion der Auflagekante (12) in eine Ebene senkrecht zu einer Längsachse (L_{T}) des Trichters (11) eine in sich geschlossene mäanderförmige Kurve ist.

9. Brusthaube nach einem der Ansprüche 1 bis 8, wobei der Öffnungswinkel (γ) des Trichters (11) über seinem Umfang gleichbleibend ist.

10. Brusthaube nach einem der Ansprüche 1 bis 9, wobei der Trichter (11) steif ausgebildet ist.

11. Brusthaube nach einem der Ansprüche 1 bis 10, wobei die Auflagekante (12) eine umlaufende Auflagefläche (120) bildet, welche eine Flächenauflage des Trichters (11) auf die Mutterbrust ermöglicht.

12. Brusthaube nach einem der Ansprüche 1 bis 11, wobei die Auflagekante (12) steif ausgebildet ist.

13. Brusthaube nach einem der Ansprüche 1 bis 12, wobei der Trichter (11) und das Anschlussteil (10) einstückig ausgebildet sind.

14. Brusthaube nach einem der Ansprüche 1 bis 13, wobei der Trichter (11) auf seiner äusseren Oberfläche mindestens ein haptisches Element (13) aufweist, welches sich von der übrigen äusseren Oberfläche des Trichters (11) unterscheidet.

15. Brusthaube für eine Brustpumpe zum Abpumpen von menschlicher Muttermilch, wobei die Brusthaube einen Trichter (11) zur Auflage auf die menschliche Mutterbrust und ein Anschlussteil (10) aufweist, wobei sich der Trichter zur Mutterbrust hin erweitert und mindestens eine Brustwarze und eine die Brustwarze umgebende Areola der Mutterbrust umschliesst, wobei der Trichter (11) ein brustnahes Ende aufweist, welches eine Auflagekante (12) zur Kontaktierung der Mutterbrust bildet, und wobei die Brusthaube einen Durchgang (14) aufweist, welcher sich vom brustnahen Ende des Trichters (11) bis zum brustfernen Ende des Anschlussteils (10) erstreckt und welcher zum Anlegen eines Unterdrucks an die Mutterbrust und zum Abfliessen abgepumpter Muttermilch dient, **dadurch gekennzeichnet, dass** der Trichter (11) ein gerader Kegelstumpf ist und dass die Auflagekante (12) des Trichters (11) durch eine nicht in einer Ebene liegende Raumkurve gebildet ist.
